(19) Europäisches Patentamt
European Patent Office
Office européen des brevets

(11) Publication number: **0 368 421 B1**

(12) **EUROPEAN PATENT SPECIFICATION**

(45) Date of publication of patent specification: **15.06.94**

(51) Int. Cl.5: **C07C 43/215**, C07C 41/16, C07D 303/27, C08G 59/20

(21) Application number: **89202835.8**

(22) Date of filing: **07.11.89**

(54) **Process for the manufacture of polyglycidylethers.**

(30) Priority: **10.11.88 GB 8826323**

(43) Date of publication of application:
**16.05.90 Bulletin 90/20**

(45) Publication of the grant of the patent:
**15.06.94 Bulletin 94/24**

(84) Designated Contracting States:
**BE CH DE ES FR GB IT LI NL**

(56) References cited:
**EP-A- 0 155 238**
**DE-B- 1 916 174**
**US-A- 3 218 370**

**CHEMICAL ABSTRACTS, vol. 103, no. 8, 26th August 1985, page 12, abstract no.54616v, Columbus, Ohio, US; & JP-A-59 189 111**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Den Haan, Klaas Hendrik**
**Badhuisweg 3**
**NL-1031 CM Amsterdam(NL)**

EP 0 368 421 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention relates to a novel process for the manufacture of poly(β-hydrocarbylglycidyl)ethers of polyhydric compounds. More particularly, the invention relates to a novel process for the manufacture of poly(β-hydrocarbylallyl)ethers of polyhydric compounds, and the subsequent epoxidation thereof. Moreover, the invention relates to the use of poly(β-hydrocarbylglycidyl)ethers of polyhydric compounds, prepared according to the present invention. The polyhydric starting material is preferably an aromatic polyalcohol that may, or may not be a condensed ring system, optionally substituted by otherwise inert substituents. However, although less preferred, aliphatic poly-alcohols may be used as starting material as well.

Polyglycidylethers (of said polyhydric compounds) are widely used as adhesives in the lacquer industry, as starting materials for coatings, and also as thermosets in the automotive industries.

In certain appliances, the polyglycidylethers are required to have lengthy hydrocarbon branches (eg., as internal plasticiser). Therefore, the urge exists to prepare β-glycidyl substituted polyglycidylethers (i.e. having substituents attached at the β carbon atom in the glycidyl radical).

From DE-B-1,916,174 and EP-A-0,155,238 a process for preparing poly(β-methylglycidyl)ether of bisphenol-A (BPA) is known, that is conducted by condensation of BPA with a stoichiometric excess of β-methylepichlorhydrin in the presence of an alkali metal hydroxide. This synthesis has the drawback that the electron-withdrawing character of the β-substituent negatively influences the reaction rate, and furthermore leads to products having a high chlorine-impurity content. Also, the availability of the starting β-substituted glycidyl compound is inadequate for commercial production.

In Chemical Abstract 103, 54617v (Jpn Kokai Jp-60-44,518 in the name of Diacel Chemical Industries, Ltd.) a process is disclosed for the preparation of epoxy resins that have a substituent attached on the β carbon of the glycidyl radical. In order to prepare the latter components, β-substituted allyl ethers are epoxidised using organic peracids (peracetic acid) in liquid phase (ethylacetate). Accordingly, a methallyl-etherified novolak was prepared by etherifying methallyl chloride with a novolak in a medium comprising an organic solvent (methylisobutyl ketone) and water, in the presence of tetrabutylammonium bromide and sodium hydroxide. Whilst the resins so prepared have very little chlorine impurities, it is a drawback of the known process that undesirable by-products are formed in which the allyl radical is directly attached to the aromatic nucleus. These by-products are, because of their conjugated double bond systems, very reactive and unpredictable upon epoxidation. Moreover, after epoxidation, the by-products will result in a material exhibiting a functionallity higher than wanted.

Consequently, the Applicants envisage to prepare poly(β-hydrocarbylglycidyl)ethers of polyhydric compounds, that are low in chlorine and by-product content.

This is achieved by providing a process for the manufacture of a poly(β-hydrocarbylallyl)ether of a polyhydric compound, which comprises contacting at an elevated temperature an allyl halide of formula I,

$H_2C=CR-CH_2X$ I

wherein R is a hydrocarbyl radical and X is a halogen atom, with a polyhydric compound in the presence of a medium comprising water and an organic solvent, a catalyst, and a base, characterised in that the base has a pKb of more than 4, comprises a monovalent anion, and is selected from bases that are capable of being transformed under the reaction conditions to compounds that will be liberated from the reaction system.

Although the process of the invention is suitable for any polyhydric compound, preference is given to aromatic compounds having 2 to 6 hydroxy groups. Of commercial interest and very suitable, are tetraphenols as disclosed in US-A-4,415,725, especially tetraphenols of formula II,

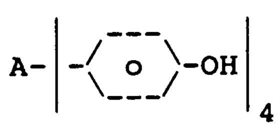

II

wherein A is a tetravalent hydrocarbyl radical having 2 to 12 carbon atoms; and bisphenols of formula III,

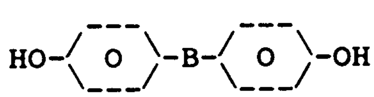

III

wherein B is divalent hydrocarbyl radical having 1 to 8 carbon atoms. Most preferred polyhydric compounds are 2,2-di(p-hydroxyphenyl)propane (BPA), and 1,1,5,5-tetra(p-hydroxyphenyl)pentane.

Preferably the base comprises a monovalent anion selected from $HCO_3^-$ , $HS^-$, or $HSO_3^-$ , and a counterion selected from alkaline, or alkaline earth metal cations.

The elevated temperature may be from 34 to 100°C, preferably from 56 to 100°C.

The hydrocarbyl radical of the allyl halide of formula I suitably is an alkyl group having from 1 to 9, more suitably 1 to 6 carbon atoms. The alkyl group may be substituted by inert substituents, like cycloalkyl or aryl groups. Most preferred allyl halides of formula I are the methallyl and ethallyl halides. The halogen atom of the allyl halide of formula I is preferably a chlorine atom.

The etherification reaction is performed in a medium comprising water and a organic solvent, i.e. a phase-transfer catalysed system. The organic phase is a solvent selected from ketones such as acetone, methylethyl ketone, or methylisobutyl ketone; aromatic compounds such as benzene, toluene, or xylene; or mixtures thereof. Most preferred solvents are toluene and methylethyl ketone.

A suitable catalyst is represented by formula IV,

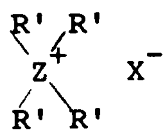

IV

wherein Z is an ammonium or phosphonium ion, $X^-$ is a halide or $OH^-$, and each R′ independently is an alkyl or arylalkyl radical having 1 to 16 carbon atoms. Preferred catalysts are tetra(n-butyl)ammonium hydroxide, and tetra(n-butyl)ammonium chloride.

Epoxidation of the poly(β-hydrocarbylallyl)ether may be accomplished by any known oxidation reaction, however oxidation by peracids in a suitable organic solvent is preferred. Very suitable peracids are metachloroperbenzoic acid and peracetic acid. The poly(β-hydrocarbylglycidyl)ethers thus prepared are remarkably pure as compared to those prepared in the prior art, and are therefore very suitable for appliances in need of highly pure substituted glycidylethers of polyhydric compounds. Moreover, the present process results in the hitherto unknown classes of compounds tetra(β-methallyl)ethers and tetra(β-methylglycidyl)ethers of α,α,Ω,Ω-tetra(p-hydroxyphenyl)alkanes, in which the alkanes have 2 to 12 carbon atoms.

In the examples below, the process for the manufacture of a poly(β-hydrocarbylallyl)ether, and subsequent poly(β-hydrocarbylglycidyl)ether of a polyhydric compound is illustrated. In the examples below the percentage in parentheses is the yield, based on the theoretical yield.

Example 1

A 3 l reactor was charged with 228.7 g of BPA (1 mole), 221.7 g of $NaHCO_3$ (2.6 mole), 16.7 g tetra(n-butyl)ammonium hydroxide (40% in $H_2O$; 0.06 mole), 500 ml of methylethyl ketone and 300 ml of $H_2O$, and warmed with stirring until the mixture refluxed gently. To this boiling mixture 250 ml of 2-methallyl chloride (2.5 mole) was added in 30 minutes. The reflux was maintained until no more $CO_2$ was released. The excess of methallyl chloride was distilled off, and 500 ml toluene was added. In order to free the product of the catalyst and salt, the toluene solution was washed three times with 500 ml of $H_2O$. Unreacted BPA and mono allyl-etherified BPA was removed by a wash with 250 ml of Claisen Alkali and subsequent washings with water until the separated water layer reacted neutral. After evaporation of the solvent 333.9 g of di(β-methallyl)ether of BPA (99%) with a purity of 97% (HPLC) was isolated. The chlorine content was 12.3 ppm.

Example 2

A 5 l reactor was charged with 708.6 g of BPA (3.1 mole), 1054 ml of freshly distilled 2-methallyl chloride (10.7 mole), 680.6 g of $NaHCO_3$ (8.1 mole), 31.0 g of tetra(n-butyl)ammonium chloride (0.1 mole), 1250 ml of methylethyl ketone and 1500 ml of $H_2O$. The mixture was warmed with stirring and refluxed. After workup as described in example 1 946.3 g of di(β-methallyl)ether of BPA (93%) with a purity of 98% (HPLC) was isolated. The chlorine content was 24 ppm.

Example 3, comparison

The process of example 1 was repeated, however 145.2 g of NaOH (2.2 mole) was used instead of the $NaHCO_3$. After workup as described above 276.8 g of di(β-methallyl)ether of BPA (82%) with a purity of 75% (HPLC) was isolated. The chlorine content was 5 ppm.

Example 4

440.2 g of 1,1,5,5 tetra(p-hydroxyphenyl)pentane (TPP) (1 mole) was dissolved in 800 ml methylethyl ketone and mixed with 1400 ml of freshly distilled 2-methallyl chloride (14.2 mole), 1000 ml of $H_2O$, 890.3 g of $NaHCO_3$ (10.6 mole) and 60.8 g of tetra(n-butyl)ammonium chloride (0.2 mole). The mixture was warmed with stirring and refluxed. After workup as described in example 1, 570.7 g of tetra(β-methallyl)ether of TPP (87%) was isolated.

Example 5, epoxidation

36.2 g of di(β-methallyl)ether of BPA (0.1 mole; example 1) was reacted with 24.2 g of meta-chlorperbenzoic acid in 250 ml diethylether at ambient temperature. After a washing (100 ml 1.0 n NaOH; 100 ml of a 5% $NaH_2PO_4$; and twice 100 ml of $H_2O$), the solvent was evaporated in vacuum. 36.5 g of di(β-methylglycidyl)ether of BPA (91%) was obtained having an epoxy group content (EGC) of 5.17 mole/kg (95.2%), i.e. an epoxy equivalent weight (EEW) of 193.

Example 6

8.4 g of di(β-methallyl)ether of BPA (25 mmole), 20.6 g of peracetic acid (36%, 97 mmole) and 60 g of $K_2HPO_4$ (344 mmole) were stirred in refluxing $CH_2Cl_2$. After workup as described above, 6.1 g of di(β-methylglycidyl)ether of BPA (66.6%) was obtained having a EGC of 5.34 mole/kg (98.2%).

Example 7

99.3 g of tetra(β-methallyl)ether of TPP (0.15 mole; example 4) was dissolved in 750 ml of toluene and warmed to 70 °C. To this solution a mixture of 41.4 g of formic acid (0.90 mole) and 78.9 g of $H_2O_2$ (65%, 1.46 mole) was added in 75 minutes on which the temperature rose to 85 °C. After a subsequent reaction time of 60 minutes 126.0 g of $Na_2SO_3$ was added to scavenge the unreacted peroxide. The mixture was filtered and washed with 500 ml of $H_2O$ containing 17.6 g of $K_2HPO_4$ (0.10 mole) followed by two washings with 500 ml of $H_2O$. After drying the toluene solution, the solvent was distilled off and 87.5 g of tetra(β-methylglycidyl)ether of TPP (81%) was obtained having an EGC of 4.41 mole/kg (79.4%).

Example 8, evaluation

15.6 g of di(β-methylglycidyl)ether of BPA (product of example 5) was mixed at 95 °C with 4.0 g of diamino diphenyl methane, degassed at 80 °C and 1 mm Hg 0.133 kPa for 3 minutes, and consequently cured by heating for 1 hour at 80 °C, 1 hour at 150 °C, 1 hour at 175 °C, and thereafter gently letting it cool down. $T_g$ (Tan Delta) is 138.5 °C, and the gel time is 385 seconds at 150 °C.

Example 9

Tetra(β-methylglycidyl)ether of TPP, when mixed with it's precursor in a 1 to 1 equivalent ratio, has a gel time of 320 seconds at 180 °C in a 50% methylethyl ketone solution.

## Claims

**1.** A process for the manufacture of a poly(β-hydrocarbylallyl)ether of a polyhydric compound, which comprises contacting at an elevated temperature an allyl halide of formula I,

$H_2C=CR\text{-}CH_2X$    I

wherein R is a hydrocarbyl radical and X is a halogen atom, with a polyhydric compound in the

presence of a medium comprising water and an organic solvent, a catalyst, and a base, characterised in that the base has a pKb of more than 4, comprises a monovalent anion, and is selected from bases that are capable of being transformed under the reaction conditions to compounds that will be liberated from the reaction system.

**2.** A process as claimed in claim 1, characterised in that the polyhydric compound is an aromatic polyalcohol having 2 to 6 hydroxy groups.

**3.** A process as claimed in claim 1, characterised in that the polyhydric compound is a tetraphenol of formula II,

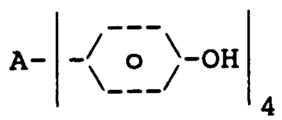

II

wherein A is a tetravalent hydrocarbyl radical having 2 to 12 carbon atoms.

**4.** A process as claimed in claim 1, characterised in that the polyhydric compound is a bisphenol of formula III,

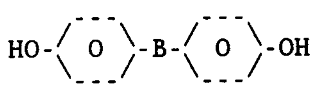

III

wherein B is divalent hydrocarbyl radical having 1 to 8 carbon atoms.

**5.** A process as claimed in any one of claims 1 to 4, characterised in that the base comprises a monovalent anion selected from $HCO_3^-$ , $HS^-$, or $HSO_3^-$ , and a counterion selected from the alkaline metal, or alkaline earth metal cations.

**6.** A process as claimed in any one of claims 1 to 5, characterized in that the hydrocarbyl radical is an optionally substituted alkyl group having 1 to 6 carbon atoms.

**7.** A process as claimed in claim 6, characterized in that the allyl halide of formula I is a methallyl or ethallyl halide.

**8.** A process as claimed in claim 6, characterized in that the polyhydric compound is 2,2-di(p-hydroxyphenyl)propane, or 1,1,5,5-tetra(p-hydroxyphenyl)pentane.

**9.** A process as claimed in any one of the claims 1 to 8, characterized in that the catalyst is represented by formula IV,

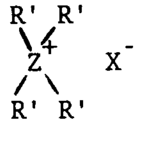

IV

wherein Z is an ammonium or phosphonium ion, $X^-$ is a halide or $OH^-$, and each R' independently is an alkyl or arylalkyl radical having 1 to 16 carbon atoms.

**10.** A process as claimed in any one of the claims 1 to 9 followed by an epoxidation step.

## Patentansprüche

1. Ein Verfahren zur Herstellung eines Poly($\beta$-kohlenwasserstoffallyl)ethers einer mehrwertigen Verbindung, umfassend das in-Berührung-bringen bei einer erhöhten Temperatur eines Allylhalogenids der Formel I,

   $H_2C=CR\text{-}CH_2X$ I

   in der R ein Kohlenwasserstoffrest und X ein Halogenatom ist, mit einer mehrwertigen Verbindung in Gegenwart eines Mediums, umfassend Wasser und ein organisches Lösungsmittel, eines Katalysators und einer Base, dadurch gekennzeichnet, daß die Base einen pKb-Wert von mehr als 4 aufweist, ein einwertiges Anion umfaßt und aus den Basen ausgewählt ist, die unter den Reaktionsbedingungen zu Verbindungen umgewandelt werden können, die aus dem Reaktionssystem freigesetzt werden.

2. Ein Verfahren, wie in Anspruch 1 beansprucht, dadurch gekennzeichnet, daß die mehrwertige Verbindung ein aromatischer Polyalkohol mit 2 bis 6 Hydroxygruppen ist.

3. Ein Verfahren, wie in Anspruch 1 beansprucht, dadurch gekennzeichnet, daß die mehrwertige Verbindung ein Tetraphenol der Formel II ist,

   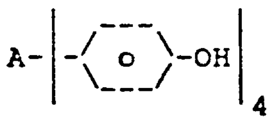

   II

   in der A ein vierwertiger Kohlenwasserstoffrest mit 2 bis 12 Kohlenstoffatomen ist.

4. Ein Verfahren, wie in Anspruch 1 beansprucht, dadurch gekennzeichnet, daß die mehrwertige Verbindung ein Bisphenol der Formel III ist,

   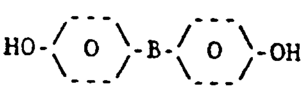

   III

   in der B ein zweiwertiger Kohlenwasserstoffrest mit 1 bis 8 Kohlenstoffatomen ist.

5. Ein Verfahren, wie in irgendeinem der Ansprüche 1 bis 4 beansprucht, dadurch gekennzeichnet, daß die Base ein einwertiges Anion, ausgewählt aus $HCO_3^-$, $HS^-$ oder $HSO_3^-$, und ein Gegenion, ausgewählt aus Alkalimetall- oder Erdalkalimetallkationen, umfaßt.

6. Ein Verfahren, wie in irgendeinem der Ansprüche 1 bis 5 beansprucht, dadurch gekennzeichnet, daß der Kohlenwasserstoffrest eine gegebenfalls substituierte Alkylgruppe mit 1 bis 6 Kohlenstoffatomen ist.

7. Ein Verfahren, wie in Anspruch 6 beansprucht, dadurch gekennzeichnet, daß das Allylhalogenid der Formel I ein Methallyl- oder ein Ethallylhalogenid ist.

8. Ein Verfahren, wie in Anspruch 6 beansprucht, dadurch gekennzeichnet, daß die mehrwertige Verbindung 2,2-Di(p-hydroxyphenyl)propan oder 1,1,5,5-Tetra(p-hydroxyphenyl)pentan ist.

9. Ein Verfahren, wie in irgendeinem der Ansprüche 1 bis 8 beansprucht, dadurch gekennzeichnet, daß der Katalysator durch die Formel IV dargestellt ist,

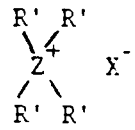

IV

in der Z ein Ammonium- oder Phosphoniumion, $X^-$ ein Halogenid oder $OH^-$, und jedes R' unabhängig ein Alkyl- oder Arylalkylrest mit 1 bis 16 Kohlenstoffatomen ist.

**10.** Ein Verfahren, wie in irgendeinem der Ansprüche 1 bis 9 beansprucht, gefolgt von einem Epoxidierungsschritt.

**Revendications**

**1.** Procédé de fabrication d'un poly($\beta$-hydrocarbylallyl)éther d'un composé possédant plusieurs fonctions hydroxy, qui comprend la mise en contact, à une température élevée, d'un halogénure d'allyle de formule I,

$H_2C=CR\text{-}CH_2X$ I

dans laquelle R est un radical hydrocarbyle et X est un atome d'halogène, avec un composé à plusieurs fonctions hydroxy, en présence d'un milieu comprenant de l'eau et un solvant organique, d'un catalyseur et d'une base, caractérisé en ce que la base possède un pKb supérieur à 4, comprend un anion monovalent et est choisie parmi les bases qui sont capables de se transformer, dans les conditions de la réaction, en composés qui seront libérés du système réactionnel.

**2.** Procédé selon la revendication 1, caractérisé en ce que le composé à plusieurs fonction hydroxy est un polyol aromatique comportant 2 à 6 groupes hydroxy.

**3.** Procédé selon la revendication 1, caractérisé en ce que le composé à plusieurs fonctions hydroxy est un tétraphenol de formule II,

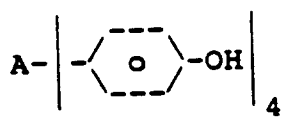

II

dans laquelle A est un radical hydrocarbyle tétravalent comportant 2 à 12 atomes de carbone.

**4.** Procédé selon la revendication 1, caractérisé en ce que le composé à plusieurs fonctions hydroxy est un bisphenol de formule III,

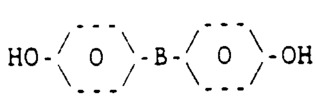

III

dans laquelle B est un radical hydrocarbyle divalent comportant 1 à 8 atomes de carbone.

**5.** Procédé selon l'une quelconque des revendications 1 à 4, caractérisé en ce que la base comprend un anion monovalent choisi parmi $HCO_3^-$, $HS^-$ ou $HSO_3^-$, et un contre-ion choisi parmi les cations de métaux alcalins ou de métaux alcalino-terreux.

**6.** Procédé selon l'une quelconque des revendications 1 à 5, caractérisé en ce que le radical hydrocarbyle est un groupe alkyle éventuellement substitué comportant 1 à 6 atomes de carbone.

**7.** Procédé selon la revendication 6, caractérisé en ce que l'halogénure d'allyle de formule I est un halogénure de méthallyle ou d'éthallyle.

**8.** Procédé selon la revendication 6, caractérisé en ce que le composé à plusieurs fonctions hydroxy est le 2,2-di(p-hydroxyphényl)propane ou le 1,1,5,5-tétra(p-hydroxyphényl)pentane.

**9.** Procédé selon selon l'une quelconque des revendications 1 à 8, caractérisé en ce que le catalyseur est représenté par la formule IV,

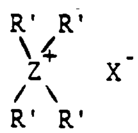

IV

dans laquelle Z est un ion ammonium ou phosphonium, $X^-$ est un halogénure ou $OH^-$, et chaque R' représente indépendamment un radical alkyle ou arylalkyle comportant 1 à 16 atomes de carbone.

**10.** Procédé selon l'une quelconque des revendications 1 a 9, suivi d'une étape d'époxydation.